(19) Europäisches Patentamt
European Patent Office
Office européen des brevets

(11) Numéro de publication: **0 469 683 A1**

# (12) DEMANDE DE BREVET EUROPEEN

(21) Numéro de dépôt: **91201972.6**

(22) Date de dépôt: **30.07.91**

(51) Int. Cl.[5]: **C07D 295/08**, A61K 31/495, C07D 295/12, C07D 295/02, C07C 217/74, C07C 211/42, C07C 215/42, C07D 333/20, C07D 317/70, A61K 31/535, A61K 31/135

(30) Priorité: **03.08.90 FR 9009996**

(43) Date de publication de la demande: **05.02.92 Bulletin 92/06**

(84) Etats contractants désignés: **AT BE CH DE ES FR GB IT LI LU NL SE**

(71) Demandeur: **LABORATOIRES LUCIEN, Société Anonyme :**
**B.P. 29**
**F-92702 Colombes, Cédex(FR)**

(72) Inventeur: **Bonnet-Delpon, Danièle**
**125, rue de la Réunion**
**F-75020 Paris(FR)**
Inventeur: **Begue, Jean-Pierre**
**241 rue de Charenton**
**F-75012 Paris(FR)**
Inventeur: **Charpentier-Morize, Micheline**
**10, allée Diane de Poitiers**
**F-75019 Paris(FR)**
Inventeur: **Gilbert, Huguette**
**12, Impasse du Clos Thiron, Moracez**
**F-28630 Chartres Cedex(FR)**
Inventeur: **Mathe, Laurence**
**CNRS-Cercoa, 2, Rue Henri Dunant**
**F-94320 Thiais(FR)**

(74) Mandataire: **Poidatz, Emmanuel**
**Cabinet M. SABATIER 83, Avenue Foch**
**F-75116 Paris(FR)**

## (54) Dérivés d'amino-4-méthyl-1-tétralines, leur préparation et leur application en thérapeutique.

(57) Dérivés d'amino-4-méthyl-1 tétralines de formule générale (I) dans laquelle X représente un noyau aromatique, notamment phényle, naphtyle, $\alpha$ ou $\beta$-thiényle, pouvant porter un à deux substituants choisis parmi halogéno, hydroxy, alcoxy en $C_1$ à $C_8$ trifluorométhyle; $R_1$ représente un atome d'hydrogène ou un halogène, un groupe hydroxy, un groupe alcoxy en $C_1$ à $C_8$, un groupe alkyle linéaire ou ramifié en $C_1$ à $C_4$ éventuellement halogéné, occupant l'une des positions 5, 6 ou 7, ou encore un groupe méthylènedioxy occupant les positions 5 et 6 ou 6 et 7; $R_2$ représente un atome d'hydrogène ou d'halogène, un groupe hydroxy, un groupe alcoxy en $C_1$ à $C_8$, un groupe alkyle linéaire ou ramifié en $C_1$ à $C_4$ éventuellement halogéné, occupant l'une des autres positions 5, 6 ou 7; et $R_3$ et $R_4$ représentent chacun un atome d'hydrogène, un groupe alkyle linéaire ou ramifié en $C_1$ à $C_n$, n étant 2 ou plus, éventuellement halogéné, hydroxylé ou aminé, ou bien $R_3$ et $R_4$, pris ensemble avec l'atome d'azote auquel ils sont rattachés forment un hétérocycle saturé, éventuellement substitué, à cinq ou six atomes dont un ou deux sont des hétéroatomes choisis parmi azote, oxygène ou soufre.

L'invention concerne également la préparation de ces composés de formule (I) et leur application comme médicament.

EP 0 469 683 A1

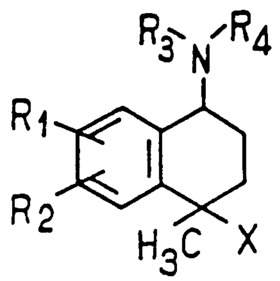

(I)

Domaine technique:

La présente invention concerne des dérivés d'amino-4-méthyl-1 tétralines, leurs procédés de préparation et leur application en thérapeutique.

Technique antérieure:

On a déjà décrit des amino-4-trifluoro-méthyl-1 tétralines présentant des activités antidépressives et analgésiques, il en est ainsi dans la demande de brevet PCT W0 89/04820.

Exposé de l'invention:

Bien que les produits décrits dans la demande précitée présentent des propriétés analgésiques satisfaisantes, le demandeur a trouvé d'autres aminotétralines offrant des propriétés pharmacologiques améliorées, notamment du point de vue toxicité et d'efficacité analgésique/antalgique, antidépressive et anti-inflammatoire et/ou avantageuses du point de vue fabrication.

A cette fin, l'invention a ainsi pour objet de nouvelles aminotétralines, et leurs sels d'addition d'acides, possédant un groupe méthyle (indifféremment identifié $CH_3$ ou $H_3C$) sur un carbone alicyclique à la place d'un atome d'hydrogène et présentant notamment des activités analgésiques/antalgiques et anti-inflammatoires.

Plus particulièrement, l'invention concerne les dérivés des amino-4-méthyl-1 tétralines répondant à la formule générale:

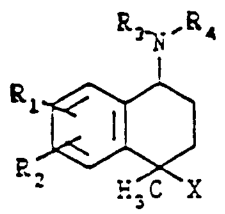

(I)

où

X représente un noyau aromatique, notamment phényle, naphtyle, $\alpha$ ou $\beta$-thiényle pouvant porter un à deux substituants choisis parmi halogéno, hydroxy, alcoxy en $C_1$ à $C_8$ trifluorométhyle;

$R_1$ représente un atome d'hydrogène ou un halogène, un groupe hydroxy, un groupe alcoxy en $C_1$ à $C_8$, un groupe alkyle linéaire ou ramifié en $C_1$ à $C_4$ éventuellement halogéné, occupant l'une des positions 5, 6 ou 7, ou encore un groupe méthylène-dioxy occupant les positions 5 et 6 ou 6 et 7;

$R_2$ représente un atome d'hydrogène ou d'halogène, un groupe hydroxy, un groupe alcoxy en $C_1$ à $C_8$, un groupe alkyle linéaire ou ramifié en $C_1$ à $C_4$ éventuellement halogéné, occupant l'une des autres positions 5, 6 ou 7; et

$R_3$ et $R_4$ représentent chacun un atome d'hydrogène, un groupe alkyle linéaire ou ramifié en $C_1$ à $C_n$, n étant 2 ou plus, éventuellement halogéné, hydroxylé ou aminé, ou bien $R_3$ et $R_4$, pris ensemble avec l'atome d'azote auquel ils sont rattachés forment un hétérocycle saturé, éventuellement substitué, à cinq ou six atomes dont un ou deux sont des hétéroatomes choisis parmi azote, oxygène ou soufre.

Parmi les composés de formule (I) définis ci-dessus, une classe préférée de composés comprend les dérivés dans lesquels X est un noyau phényle pouvant porter un à deux substituants choisis parmi les radicaux halogéno, hydroxy, alcoxy en $C_1$ à $C_8$.

Les composés de formule (I) dans laquelle X représente un noyau phényle portant 1 à 2 atomes d'halogène (notamment en atome de fluor en position 4) ou un groupe méthoxy semblent particulièrement avantageux.

Sont également particulièrement avantageux les composés de formule (I) dans laquelle $R_1$ est un atome d'hydrogène ou un groupe tertiobutyle et $R_2$ un atome d'hydrogène. En particulier, le groupe tertiobutyle a pour effet d'accroître la lipophilie du composé.

Les composés de formule (I) possèdent une fonction amine sur le carbone numéro 4; selon la nature de $R_3$ et $R_4$ les composés de l'invention peuvent donc être des amines primaires, des amines secondaires ou des amines tertiaires, à titre d'exemples, on peut citer:

- les amines primaires dans lesquelles $R_3$ et $R_4$ représentent un atome d'hydrogène;
- les amines secondaires dans lesquelles $R_3$ représente un atome d'hydrogène et $R_4$ représente un groupe méthyle ou un groupe de formule $-(CH_2)_n-OH$ dans laquelle n est 2 ou plus;
- les amines tertiaires dans lesquelles $R_3$ et $R_4$ représentent chacun un groupe méthyle ou bien $R_3$ et $R_4$ pris ensemble avec l'atome d'azote auquel ils sont rattachés forment un groupe de formule

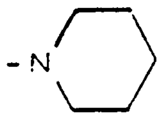 , 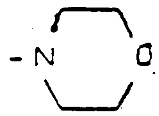 , 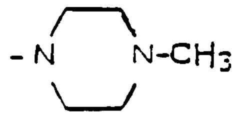 , 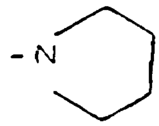 , 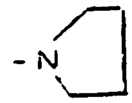

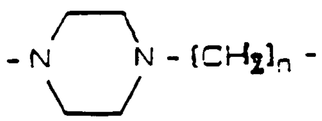 où n = 2 ou plus et/ou OH peut être sous forme ester, uréthane pour n = 2

A titre d'halogène on préfère particulièrement le fluor en premier lieu et le chlore en second lieu.

A titre d'exemples non limitatifs de composés de formule (I) qui sont utiles selon la présente invention, on peut citer les dérivés suivants et leurs chlorhydrates, qui figurent dans le tableau 1 donné ci-après.

## Tableau 1

| Composé N° | $R_2$ | $R_1$ | $R_3$ | $R_4$ | X |
|---|---|---|---|---|---|
| 1 | H | H | 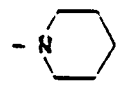 | | 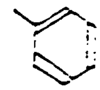 |
| 2 | H | H | $-CH_3$ | $-CH_3$ | 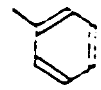 |
| 3 | H | H | H | $-CH_3$ | 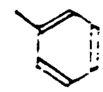 |
| 4 | H | H | 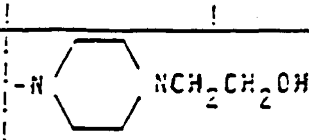 | | 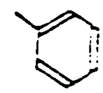 |
| 5 | H | $-OCH_3$ en position 7 | H | $CH_3$ | 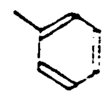 |
| 6 | H | $-OCH_3$ en position 7 | 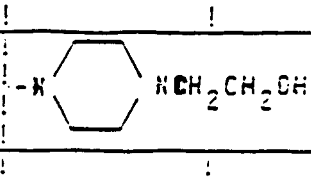 | | 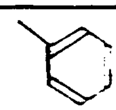 |
| 7 | H | methylene-dioxy en position 6et7 | H | $CH_3$ | 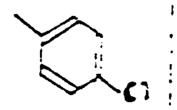 |
| 8 | H | $-OCH_3$ en position 7 | $CH_3$ | $CH_3$ | 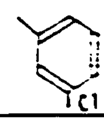 |
| 9 | H | Cl en position 7 | 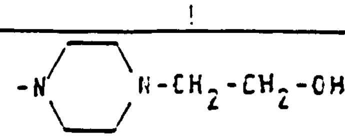 | | 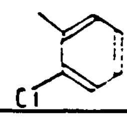 |

Tableau 1 (suite)

| Composé N° | $R_2$ | $R_1$ | $R_3$ | $R_4$ | X |
|---|---|---|---|---|---|
| 10 | Cl en position 6 | Cl en position 7 | H | H | 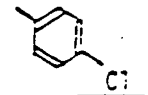 |
| 11 | H | $-OCH_3$ en position 7 | H | $CH_3$ | 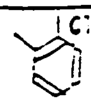 |
| 12 | H | H | H | $-CH_2-CH_2-OH$ | 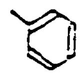 |
| 13 | H | H | 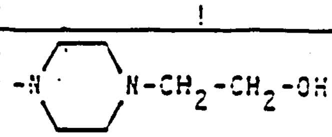 | | 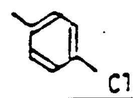 |
| 14 | H | H | H | $CH_3$ | 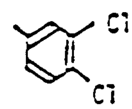 |
| 15 | H | $-OCH_3$ en position 7 | H | $-CH_3$ | 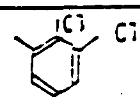 |
| 16 | H | H | $CH_3$ | $-CH_2-CH_2-OH$ | 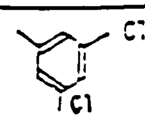 |
| 17 | H | Cl en position 7 | 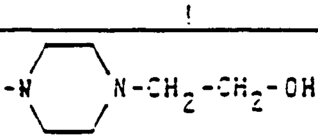 | | 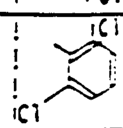 |
| 18 | H | $OCH_3$ en position 7 | H | $CH_3$ | 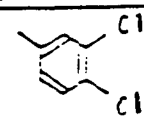 |
| 19 | H | H | 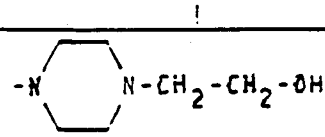 | | 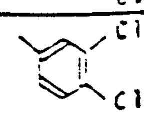 |

**Tableau 1 (suite)**

| Composé N° | $R_2$ | $R_1$ | $R_3$ | $R_4$ | X |
|---|---|---|---|---|---|
| 20 | H | H | 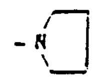 | | 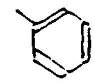 |
| 21 | H | H | 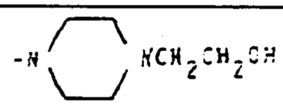 | | 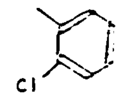 |
| 22 | H | H | 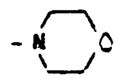 | | 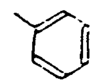 |
| 23 | H | H | H | 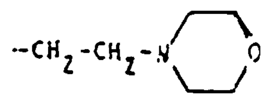 | 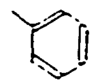 |
| 24 | H | H | H | H | 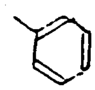 |
| 25 | H | H | H | 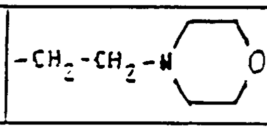 | 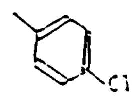 |
| 26 | H | H | 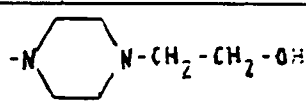 | | 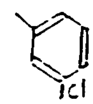 |
| 27 | H | H | 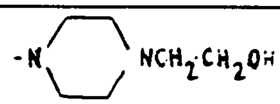 | | 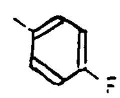 |
| 28 | H | H | 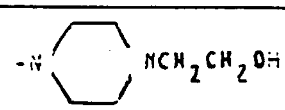 | | 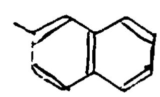 |
| 29 | H | H | 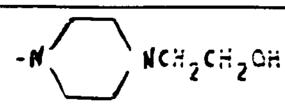 | | 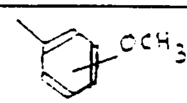 |
| 30 | H | Cl en position 7 ou 6 | 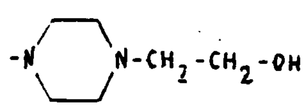 | | 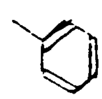 |

**Tableau 1 (fin)**

| Composé N° | R2 | R1 | R3 | R4 | X |
|---|---|---|---|---|---|
| 31 | H | H | 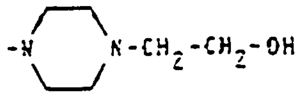 | | 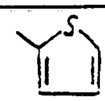 |
| 32 | H | tertio-butyl en position 7 | 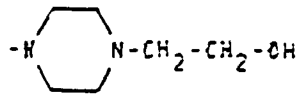 | | 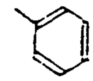 |
| 33 | H | H | 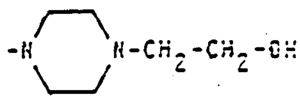 | | 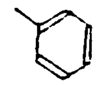 |
| 34 | H | $-OCH_3$ en position 7 | 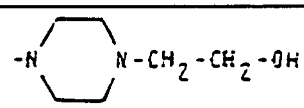 | | 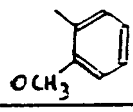 |
| 35 | H | $-OCH_3$ en position 7 | 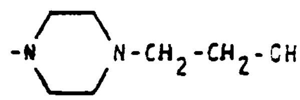 | | 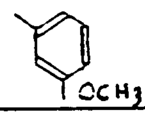 |
| 36 | H | tertio-butyl en position 7 | 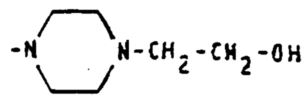 | | 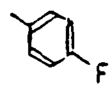 |
| 37 | H | tertio-butyl en position 7 | 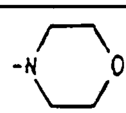 | | 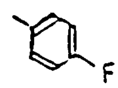 |
| 38 | H | H | H | $CH_3$ | 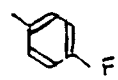 |
| 39 | H | tertio-butyl en position 7 | 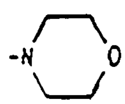 | | 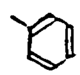 |

On remarque du tableau 1 que dans les cas où le groupe $NR_3R_4$ se présente sous la forme d'un groupement pipérazine (entre autres, les composés 26 à 36), la substitution sur l'hétérocycle est réalisée au niveau du deuxième hétéroatome d'azote; plus particulièrement le groupement apparaît sous la forme pipérazine-éthanol:

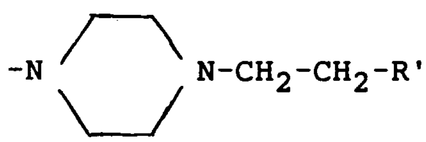

avec R' = OH

le radical OH de la fonction étant non bloqué.

Bien entendu, sans sortir du cadre de l'invention, le radical OH de la fonction alcool liée à l'amine tertiaire peut être bloqué soit sous forme d'ester, soit sous forme d'uréthanne.

A titre d'exemple, on peut citer pour les esters, les composés où

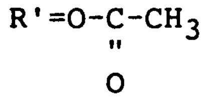

(radical acétyle), en particulier le composé 36-a correspondant au composé 36 et étudié ultérieurement du point de vue pharmacologique.

En ce qui concerne les uréthannes, on peut citer:

i) les composés où

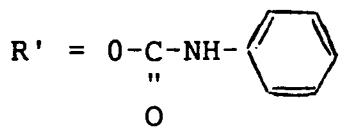

(radical phényl-uréthanne), en particulier les composés 27-p et 36-p correspondant respectivement aux composés 27 et 36,

ii) les composés où

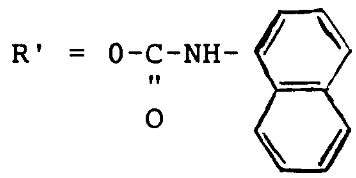

(radical naphtyluréthanne), en particulier les composés 27-n et 36-n correspondant respectivement aux composés 27 et 36.

Les synthèses décrites ci-après conduisent à un mélange racémique de deux diastéréoisomères a et b que l'on peut séparer par des procédés classiques de chromatographie ou de cristallisation fractionnée, chaque diastéréoisomère étant susceptible d'être dédoublé en deux isomères optiques.

Tous ces composés entrent dans le cadre de la présente invention.

Les composés de formule (I) peuvent former des sels qui font également partie de l'invention. Les sels formés de manière classique comprennent les sels d'addition d'acide que l'on forme avec divers acides minéraux et organiques par exemple les halohydrates, le sulfate, le nitrate, le tartrate, le mandelate, l'acétate, le succinate, le benzènesulfate.

Les composés de la présente invention peuvent être préparés par les divers procédés définis ci-après.

Les composés de formule I peuvent être préparés à partir des dérivés des méthyl-1-aryl-1 tétralines de formule suivante:

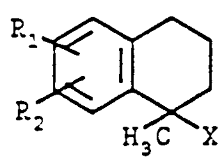

(III)

dans laquelle $R_1$, $R_2$ et X ont la même signification que dans la formule (I).

Les dérivés des méthyl-1-aryl-1 tétralines (III) peuvent le cas échéant être préparés par solvolyse en milieu acide des dérivés des diaryl-2,5 pentane ol-2 correspondants (connus) de formule (II):

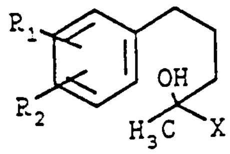

(II)

dans laquelle $R_1$, $R_2$ et X ont la même signification que dans la formule (I). Ces derniers étant obtenus classiquement à partir de l'acétophénone (substituée ou non) et du Grignard ou du lithien du dérivé de l'aryl-3-bromo-1-propane choisi.

La solvolyse, en milieu acide, des composés de formule (II) conduit aux dérivés méthyl-1-aryl-1-tétrahydronaphtalène-1,2,3,4 correspondants. Le milieu acide peut être constitué d'un acide protique, tel que l'acide sulfurique, l'acide trifluoroacétique, dans un solvant, tel que le dichlorométhane, le benzène, le toluène, l'acide trifluoroacétique. La solvolyse peut alors être effectuée au reflux du solvant ou à température ambiante, pendant une durée supérieure à 24 h, pour obtenir le composé de formule (III) correspondant. Ce dernier, après évaporation du solvant, est extrait avec de l'éther, lavé avec $NH_4OH$ et de l'eau jusqu'à neutralité, séché sur $MgSO_4$, concentré et purifié par filtration sur gel de silice (élution au pentane).

A titre de variante, le milieu acide peut également être constitué d'un acide de Lewis, $AlCl_3$ étant préféré, dans un solvant tel que $CH_2Cl_2$ (dichlorométhane), le toluène ou le benzène. La solvolyse est alors effectuée à température ambiante pour obtenir le composé de formule (III), qui est ensuite isolé comme précédemment décrit.

Un premier procédé de préparation (variante P1.1) d'un composé de formule (I) consiste à halogéner un dérivé d'une méthyl-1-aryl-1 tétraline de formule (III) dans laquelle $R_1$, $R_2$ et X ont la même signification que dans la formule (I) pour obtenir l'halogénure correspondant de formule

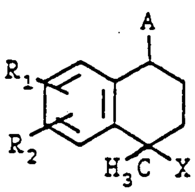

(IV)

dans laquelle $R_1$, $R_2$ et X ont la même signification que dans la formule (I) et A représente un halogène, et à faire réagir sur ce dernier une amine de formule (V):

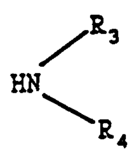

(V)

dans laquelle $R_3$ et $R_4$ ont la même signification que dans la formule (I) pour obtenir un mélange des deux diastéréoisomères du composé de formule (I) correspondant.

La réaction d'halogénation d'un dérivé d'une méthyl-1-aryl-1 tétraline (III) selon le présent procédé est avantageusement effectuée avec une halogénosuccinimide, au reflux de $CCl_4$ et en présence de péroxyde de benzoyle. La réaction entre l'halogénure (IV) et l'amine (V) est effectuée en solution organique (toluène, acétone, $CH_2Cl_2$, $CHCl_3$...) pendant une durée supérieure à 24 h à température ambiante ou quelques heures au reflux du solvant.

Selon une autre variante (P 1.2) du procédé décrit ci-dessus, la voie d'accès à l'halogénure de formule (IV) passe par une oxydation benzylique d'un dérivé de formule (III) pour obtenir la tétralone correspondante de formule (VI):

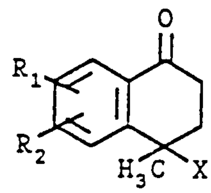

(VI)

dans laquelle $R_1$, $R_2$ et X ont la même signification que dans la formule (I).

La tétralone de formule (VI) est ensuite réduite par un hydrure, $NaBH_4$ ou $LiAlH_4$ pour obtenir le tétralol correspondant de formule (VII):

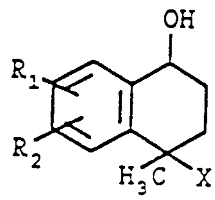

(VII)

dans laquelle $R_1$, $R_2$ et X ont la même signification que dans la formule (I).

Le tétralol de formule (VII) est ensuite substitué par un chlorure, en présence de chlorure de thionyle pour donner le composé de formule (IV). La réaction entre l'halogénure de formule (IV) et l'amine de formule (V) est effectuée en solution organique (toluène, acétone, $CH_2Cl_2$, $CHCl_3$...) pendant une durée supérieure à 24 h à température ambiante ou quelques heures au reflux du solvant.

Les composés de formule (I) peuvent être également préparés par un second procédé (P2) passant par la tétralone de formule (VI) qui est mise à réagir avec une amine de formule (V) pour obtenir un dérivé azoté (oxime si $R_3$ et $R_4$ représentent un atome d'hydrogène, imine si $R_3$ ou $R_4$ représente un atome d'hydrogène et énamine si ni $R_3$ ni $R_4$ n'est un hydrogène) que l'on réduit par un hydrure, $NaBH_4$ ou $LiAlH_4$, pour obtenir l'amino-4-méthyl-1-tétraline correspondante qui peut être une amine primaire ($R_3$ et $R_4$ représentent un atome d'hydrogène), une amine secondaire ($R_3$ ou $R_4$ représente un atome d'hydrogène) ou une amine tertiaire (ni $R_3$ ni $R_4$ ne représente un atome d'hydrogène). Le schéma réactionnel général du procédé P2 est illustré à la figure 2.

Dans les deux procédés proposés ci-dessus, les amines de formule (V)

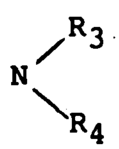

n'ont pas été particulièrement spécifiées. En ce qui concerne les amines du type tertiaire pourvues d'une fonction alcool (radical OH non bloqué), par exemple le composé pipérazine-éthanol, le blocage du radical OH peut être réalisé de la façon suivante:

i) formation d'un ester.

L'ester est obtenu par action de l'anhydride d'acide sur une amine (V) à fonction alcool en milieu organique et en présence de traces de 4-diméthylamino-pyridine à la température ambiante pendant 24 h pour donner une amine (V) ester.

ii) formation d'un uréthanne

L'uréthanne est obtenu par action d'un isocyanate sur une amine (V) à fonction alcool dans un solvant organique anhydre à température ambiante pendant 24 h.

D'autres caractéristiques et avantages de l'invention apparaîtront à la lecture de la description ci-après d'exemples donnés à titre d'illustration, de préparation de composés de formule (I) , le cas échéant en

référence aux figures présentées ci-dessous.

Présentation des figures:

Les exemples et figures ci-après sont donnés à titre d'illustration des procédés de préparation des composés, selon la présente invention:

- la figure 1 représente le schéma réactionnel général selon le premier procédé de préparation des composés de formule (I) dans ces variantes P1.1 et P1.2;
- la figure 2 représente le schéma réactionnel général selon le second procédé P2 de préparation des composés de formule (I).

Description des modes de réalisation de l'invention:

La préparation des dérivés des diaryl-2,5-pentane-ol-2 suit le protocole opératoire ci-dessous: Une solution de dérivé de l'aryl-2-bromo-1-propane choisi (0,13 mole) dans de l'éther anhydre (130 ml) est ajoutée goutte à goutte, sous agitation, à du magnésium (0,14 mole) dans de l'éther anhydre (10 ml). Chauffer sans atteindre le reflux du solvant peut aider à relancer la réaction. Après 4 h, on ajoute à la solution de Grignard obtenu, sous argon, une solution du dérivé de l'acétophénone choisi (0,11 mole) dans l'éther anhydre (110 ml). Après agitation de 2 h à température ambiante, le mélange est hydrolysé par une solution saturée en chlorure d'ammonium (150 ml). La phase aqueuse est extraite avec de l'éther. Les phases organiques combinées sont lavées avec une solution saturée en chlorure de sodium, séchées ($MgSO_4$), concentrées et purifiées (par chromatographie sur gel de silice ou par distillation).

Les autres étapes à effectuer en vue d'obtenir le composé de formule (I) sont décrites pour chacun des deux exemples suivants:

Exemple 1: Préparation du composé n° 36 par l'intermédiaire de l'halogénure correspondant (figure 1, variante P1.1)

$R_1$ représente un groupe tertiobutyle en position 7, $R_2$ représente un atome d'hydrogène, X représente un noyau phényle substitué d'un atome de fluor en position 4' et $R_3$ et $R_4$ pris ensemble, avec l'atome d'azote auquel ils sont rattachés forment un groupe de formule

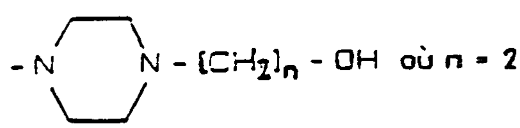

dans la formule (I).

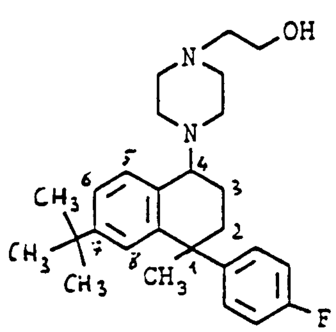

(I) n° 36

La préparation du composé de formule (I) n° 36 est réalisée par l'intermédiaire du paratertiobutyl-phényl-5-parafluoro phényl-2-pentane-ol-2 (formule II) obtenu, selon le protocole opératoire donné ci-dessus, à partir du Grignard du paratertiobutylphényl-3-bromo-1-propane et de la parafluoroacétophénone, avec un rendement de 75% après purification par chromatographie ($SiO_2$, pentane/éther 95:5). La solvolyse est réalisée sur 30,8 g (98 mmoles) de paratertiobutylphényl-5-parafluoro-phényl-2-pentane-ol-2 (formule II) en solution dans 200 ml de $CH_2Cl_2$ en présence de 4 ml (71 mmoles) d'$H_2SO_4$ concentré, sous agitation et

à température ambiante pour obtenir 21,15 g (71 mmoles) de méthyl-1-tertiobutyl-7-parafluorophényl-1-tétrahydronaphtalène-1,2,3,4, (tétraline de formule III), soit un rendement de 73%. On ajoute à une solution de 3 g (10,1 mmoles) de tétraline, préparée ci-dessus, dans 40 ml de $CCl_4$, 400 mg de péroxyde de benzoyle et 1,89 g (10,6 mmoles) de N-bromosuccinimide. Après 45 minutes de reflux sous agitation magnétique, la solution refroidie est concentrée sous vide. Le résidu, repris par du pentane, est rapidement filtré sur un verre fritté recouvert d'un coton. Après évaporation du solvant, on obtient 4,3 g de bromure que l'on utilise tel quel. Ce bromure, dissous dans 40 ml d'acétone, est laissé au reflux avec 3,3 g (25 mmoles soit 2,5 la concentration molaire équivalente) de pipérazine éthanol (composé de formule V) pendant 3 heures. Le mélange réactionnel est extrait 2 fois par de l'acide chlorhydrique à 10%. Les fractions acides rassemblées sont extraites à l'éther, filtrées et traitées par $NH_4OH$ à 10%. La base ainsi libérée est extraite par de l'éther; après plusieurs lavages à l'eau pour éliminer l'excès de pipérazine éthanol, la solution est séchée ($MgSO_4$) puis concentrée sous vide. On obtient après purification par chromatographie ($SiO_2$, élution à l'éther) 2,1 g (5 mmoles) d'un mélange 50/50 des deux diastéréoisomères. Ces deux diastéréoisomères peuvent être séparés par chromatographie ou par recristallisation de sels.

Caractéristiques du composé n° 36

- analyse du chlorhydrate (2HCl):
  Formule brute: $C_{27}H_{39}N_2O\ F\ Cl_2$
  Poids moléculaire: 497,58

| | | | |
|---|---|---|---|
| Cal % | C: 65,17 | H: 7,92 | N: 5,63 |
| Tr % | C: 65,24 | H: 8,00 | N: 5,59 |

- analyse de sa base par RMN ($CDCl_3$): $\delta$ (ppm), isomère A/isomère B

$^1H$ : 6.7-7.6 (m,7H,2$\phi$) ; 3.7 (m, 1H, CH-N) ; 3.5 (m, 3H, $CH_2OH$) ; 2.5 (m,10H) ; 2.0-1.7 (m, 4H) ; 1.6/1.56 (s, 3H, $CH_3$) ; 1.15/1.05 (s, 9H, tBu).

$^{19}F$ : -118 (réf. $CFCl_3$)

$^{13}C$ : 17.1/18.0 ; 30.9/29.4 ; 31.2/31.1 ; 34.3/34.1 ; 39.2/40.1 ; 42.3/42.8 ; 47.9 ; 53.61/53.65 ; 57.7/57.74 ; 59.58/59.64 ; 62.8/62.2 ; 114.16/114.23 (d, J=20.8 Hz, $C_3$'et $C_5$') ; 123.0/122.6 ; 125.1/126 ; 127.7/127.1 ;
128.7 (d, J=7.4 Hz, $C_2$'et $C_6$') ; 134.9/134.3 ; 143.4/145.1 ; 147.02/146.99 (d, J=3.15 Hz, $C_1$') ; 149.2/148.8 ; 160.61/160.65 (d, J=244 Hz, $C_4$').

Exemple 2: Préparation du composé n° 29 par l'intermédiaire de l'halogénure correspondant (figure 1, variante P1.2)

$R_1$ et $R_2$ représentent un atome d'hydrogène, X représente un noyau phényle substitué d'un groupe méthoxy en position 4', et $R_3$ et $R_4$ pris ensemble avec l'atome d'azote auquel ils sont rattachés forment un groupe de formule

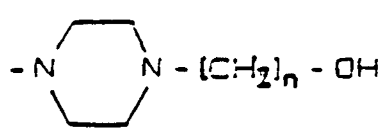 où n = 2

dans la formule (I).

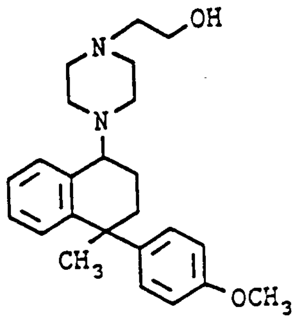

(I) n° 29

La préparation du composé de formule (I) n° 29 est réalisée par l'intermédiaire du phényl-5-paraméthoxyphényl-2-pentane-ol-2 (formule II) obtenu selon le protocole opératoire donné ci-dessus à partir du Grignard du phényl-3-bromo-1-propane et de la paraméthoxyacétophénone, avec un rendement de 75%, sans purification. La solvolyse est réalisée sur 19,4 g (72 mmoles) d'alcool obtenu précédemment dans 60 ml de benzène, en présence de 1,1 ml (20 mmoles) d'$H_2SO_4$ concentré, sous agitation et au reflux du solvant (ballon muni d'un dispositif Dean-Stark pour obtenir une distillation azéotropique) pendant 12 h. On obtient 10,5 g (42 mmoles) de méthyl-1-paraméthoxyphényl-1-tétrahydronaphtalène-1,2,3,4, (formule III) soit un rendement de 58%. La préparation du composé n° 29 passe par l'intermédiaire de la tétralone (formule VI) correspondante: on porte au reflux pendant 1h30 10 g (40 mmoles) de méthyl-1-paraméthoxyphényl-1-tétrahydronaphtalène-1,2,3,4 (formule III) dans 120 ml d'acétonitrile et 120 ml d'eau en présence de 9,6 g de sulfate de cuivre et 20 g de perodisulfate de potassium. Après extraction par $CH_2Cl_2$, lavage jusqu'à neutralité, la solution séchée ($MgSO_4$) est concentrée sous vide. On isole 6,7 g (25 mmoles) de la tétralone (formule VI) après purification par filtration sur gel de silice (pentane/éther), soit un rendement de 63%. Cette dernière est réduite par $LiAlH_4$, selon le protocole opératoire suivant: 6g (22,5 mmoles) de tétralone sont versés goutte à goutte sur 430 mg (11,3 mmoles) de $LiAlH_4$ en solution dans 250 ml d'éther anhydre. L'addition et la réaction se font entre O° C et -5° C pendant 2 h. En fin de réaction, l'excès d'hydrure est hydrolysé par de l'éther additionné d'eau. La phase organique est séparée, lavée à l'eau, séchée ($MgSO_4$), filtrée et concentrée pour obtenir 4,8 g (18 mmoles) de tétralol (formule VII), soit un rendement de 80% après purification par filtration ($SiO_2$, pentane/éther). 4,5 g (17 mmoles) de tétralol, obtenu précédemment, en solution dans 90 ml d'éther anhydre et 4 g (34 mmoles)de chlorure de thionyle ($SOCl_2$) sont placés sous agitation à température ambiante pendant 2 h pour donner après évaporation sous vide du solvant et de $SOCl_2$ 4,7 g (16,3 mmoles) de méthyl-1-paraméthoxyphényl-1-chloro-4-tétraline (formule IV) utilisé tel quel. Ce chlorure de formule (IV) dissous dans 70 ml de toluène est laissé en contact avec 5,5 g (42 mmoles) de pipérazine éthanol (formule V) pendant 3 jours. Après traitement acido-basique (par HCl et $NH_4OH$) décrit plus haut dans l'exemple 1, on obtient, après purification par chromatographie ($SiO_2$, $CH_2Cl_2/CH_3OH$ 90:10), 3,5 g d'un mélange 60/40 soit un rendement de 69%. Ces 2 diastéréoisomères peuvent être séparés par chromatographie ou par recristallisation de sels.

Caractérisation du composé n° 29

- analyse du chlorhydrate (2HCl):
  Formule brute: $C_{24}H_{34}N_2O_2Cl_2$
  Poids moléculaire: 453,5

| | | | |
|---|---|---|---|
| Cal%: | C: 63,56 | H: 7,57 | N: 6,18 |
| Tr%: | C: 63,67 | H: 7,49 | N: 6,12 |

- analyse de sa base par RMN ($CDCl_3$): $\delta$ (ppm), isomère A/isomère B

$^1H$ : 7.7-6.6 (m, 8H, 2$\phi$) ; 3.8 (m, 1H, CH-N) ; 3.63/3.64 (s, 3H,$OCH_3$) ; 3.5 (m, 3H,$CH_2OH$) ; 2.4 (m, 10H) ; 1.6/1.57 (s, 3H, $CH_3$) ; 2.1 à 1.4 (m, 4H).

$^{13}C$ : 17.2/18.0 ; 30.9/29.4 ; 39.1/39.9 ; 41.9/42.32 ; 47.9 ; 54.9/53.7 ; 57.8/59.7 ; 63.1/62.5 ; 112.9/113.1 ; 125.9/125.5 ; 126.5/126.3 ; 128.0/127.4 ; 128.2/128.1 ; 128.5/129.2 ; 137.9/137.4 ; 142.9/143.7 ; 144.7/146.4 ; 157.2/157.25.

Etude toxico-pharmacologique

L'étude pharmacologique des composés de l'invention a permis de mettre en évidence un effet analgésique. Les épreuves pharmacologiques réalisées ont été les suivantes:

A. Toxicité aiguë

La détermination de la mortalité chez la souris est observée à la suite de l'administration unique par voie intrapéritonéale de doses croissantes de composés à tester.

La dose limite DL100 pour les composés étudiés est supérieure ou égale à 100 mg/kg. Les doses DL0 et DL100 des composés 27, 32 et 36 sont rassemblées dans le tableau 2:

Tableau 2

| Composé | n° Dose limite en mg/kg | 0 Dose limite 100 en mg/kg |
|---|---|---|
| 27 | 100 | 200 |
| 27.p | >200 | |
| 32 | 25 | 100 |
| 36 | 50 | 100 |
| 36.a | 100 | >200 |
| 36.p | 100 | >200 |
| 36.n | >200 | |

Par rapport aux composés analogues appartenant au groupe des amino-4-trifluorométhyl-1 tétralines, la toxicité des composés selon l'invention n'est pas augmentée. Il est même à signaler que pour le composé n° 27 les doses limites DL0 et DL100 sont deux fois celles de l'amino-4-trifluorométhyl-1 tétraline correspondantes. Enfin la toxicité diminue lorsque le composé se présente sous la forme acétate ou uréthanne (voir les résultats des composés 27 et 27.p et 36, 36.a, 36.p, 36.n).

B. Activité analgésique/antalgique:

B1. Test de la plaque chauffante chez la souris.

Cet essai consiste à mesurer le temps d'apparition d'une réaction au stimulus thermoalgique chez la souris.

L'animal est placé sur une plaque métallique portée à une température constante de 56° C. Il réagit par un lèchement des pattes antérieures en un temps variant de 4 à 12 secondes. Après administration d'un analgésique, le délai d'apparition du réflexe s'allonge: on obtient une élévation de réaction du seuil.

Les composés ont été administrés par voie intrapéritonéale sous forme de chlorhydrate et testés sur les animaux 1 heure après leur administration. Les résultats sont donnés sur quelques exemples dans le tableau 3 suivant un pourcentage de variation du temps de réaction moyen après traitement par rapport à avant traitement.

Tableau 3

| Composé n° | Dose administrée | Variation du temps de réaction moyen en % |
|---|---|---|
| 27 | 15mg/kg | 29 |
| 32 | 8mg/kg | 16 |
| 36 | 8mg/kg | 9 |
| 36.a | 15mg/kg | 35 |
| 36.p (trans) | 25 mg/kg | 63* |
| 36.n (cis) | 100mg/kg | 87** |
| 39 trans | 25 mg/kg | 58 |

Ces résultats reflètent une activité analgésique/antalgique sur le système nerveux central intéressante pour le composé étudié. Toutefois le degré de signification de ces résultats par la méthode du test "t" de

Student s'est avéré insuffisant à l'exception des composés 36.p et 36.n (racémiques cis et trans).

B2. Test des crampes abdominales chez la souris

Ce test, également connu sous le nom de WRITHING TEST, utilisé pour la sélection d'analgésiques/antalgiques à effet périphérique, consiste à rechercher une éventuelle protection vis-à-vis des crampes et/ou contorsions abdominales provoquées, chez la souris, par l'injection intrapéritonéale d'acide acétique.

Les résultats de ce test exprimés en pourcentage de réduction du nombre de crampes obtenu chez les animaux traités par rapport aux témoins sont reportés au tableau 4. Les composés ont été administrés sous forme de chlorhydrate par voie intrapéritonéale 30 minutes avant l'acide acétique.

Tableau 4

| Composé n° | Dose administrée en mg/kg | Degré de protection par rapport aux témoins | Degré de signification |
|---|---|---|---|
| 27 | 4 | 49 | * |
| 27 | 8 | 66 | ** |
| 27 | 15 | 92 | *** |
| 27.p (trans) | 0,25 | 47 | * |
| 27.p (trans) | 0,5 | 58 | ** |
| 27.p (trans) | 1 | 70 | *** |
| 27.p (trans) | 2 | 68 | *** |
| 27.p (trans) | 4 | 75 | *** |
| 27.p (trans) | 8 | 92 | *** |
| 32 | 8 | 41 | * |
| 36 | 2 | 35 | * |
| 36 | 4 | 40 | * |
| 36 | 8 | 63 | *** |
| 36.a | 2 | 52 | * |
| 36.a | 4 | 66 | ** |
| 36.a | 8 | 94 | *** |
| 36.a | 15 | 85 | *** |
| 36.p (cis) | 0,5 | 61 | ** |
| 36.p (cis) | 1 | 44 | * |
| 36.p (cis) | 2 | 61 | ** |
| 36.p (cis) | 4 | 90 | *** |
| 36p. (cis) | 8 | 93 | *** |
| 36.n (cis) | 0,5 | 43 | ** |
| 36.n (cis) | 1 | 56 | *** |
| 36.n (cis) | 2 | 69 | *** |
| 36.n (cis) | 4 | 93 | *** |
| 36.n (cis) | 8 | 96 | *** |
| 39 (cis) | 0,5 | 53 | * |
| 39 (cis) | 1 | 58 | ** |
| 39 (cis) | 2 | 54 | *** |
| 39 (cis) | 4 | 63 | *** |
| 39(cis) | 8 | 89 | *** |

Le degré de signification de différences entre sujets témoins et sujets traités est donné par le test "t" de Student avec

* correspondant à p <0,05

** correspondant à p <0,01

*** correspondant à p <0,001

Nota: Pour les racémiques 27.p (cis), 36.p (trans), 36.n (trans), et 39 (trans), des tests complémentaires ont montré des résultats comparables à ceux présentés ci-dessus pour les racémiques correspondants 27.p (trans), 36.p (cis) et 39 (trans).

Il est intéressant de remarquer que le composé n° 27 montre une activité nettement améliorée par rapport à celle montrée par l'amino-4-trifluoro-méthyl-1 tétraline correspondante.

Ce test s'est également révélé satisfaisant pour le composé n° 27 en cas d'administration par voie orale avec des pourcentages de protection de 37 (*) pour une dose de 32 mg/kg et de 86 (***) pour une dose de 60 mg/kg. Ce dernier résultat est particulièrement intéressant en ce qu'il montre une activité par voie orale supérieure à celle observée pour les amino-4-trifluorométhyl-1 tétralines. De même les composés 36.a (acétate) et 36.p (racémique phényluréthanne) se sont révélés actifs par voie orale pour une dose de 16 mg/kg.

Du point de vue cinétique de l'activité antalgique, des tests de crampes abdominales ont été poursuivis dans le temps avec des relevés à 30 minutes, 1 heure, 2 heures, 4 heures et 6 heures. Les résultats de ces tests notamment au niveau de l'intérêt des composés esters et uréthannes sont donnés dans le tableau 5 ci-après:

Tableau 5

| (Degré de protection par rapport aux témoins) | | | | | | |
|---|---|---|---|---|---|---|
| Composé | Dose administrée en mg/kg | à 30´ | à 1 h | à 2 h | à 4 h | à 6 h |
| 27.p(trans) | 4 | 84*** | 68*** | 53** | 56*** | 21 |
| 36 | 8 | 95*** | 72*** | 59*** | 20 | 29 |
| 36.a | 4 | 69*** | 82*** | 60** | 58*** | 38** |
| 36.p(cis) | 4 | 89*** | 69*** | 68*** | 46** | 24 |

Il résulte des résultats de tests présentés dans les tableaux 4 et 5 que par rapport aux composés présentant le groupe pipérazine-éthanol (fonctions alcool avec radical OH non bloqué), les composés correspondants acétate, phényluréthanne et naphtyluréthanne sont plus actifs (à temps d'action égal), les composés uréthannes présentant les meilleurs résultats (composés 27.p, 36.p et 36.n). Du point de vue cinétique les composés acétate et uréthannes montrent une prolongation de la durée d'action (6 h pour l'acétate, 4 h pour les phényluréthannes au lieu de 2 h pour le composé correspondant à radical OH non bloqué).

C. Activité antidépressive

L'activité antidépressive de composés selon l'invention a été mise en évidence par l'étude du comportement du désespoir chez la souris.

Selon le principe du test, un état dépressif est induit chez la souris en l'obligeant à nager dans un

récipient cylindrique étroit d'où elle ne peut s'échapper. Après une période brève d'intense activité, la souris adopte une position immobile caractéristique, facilement identifiable. Les antidépresseurs réduisent ces moments d'immobilité.

Selon le protocole expérimental, les souris sont pré-exposées à l'eau à 25 °C pendant 15 minutes, 24 heures avant le test. Pour le test on administre le composé à étudier 30 minutes avant une exposition à l'eau de 6 minutes. Le temps total d'immobilité de la 2e à la 6e minute est mesuré. Les animaux témoins reçoivent le véhicule d'administration du produit de traitement dans les mêmes conditions que les animaux traités.

Ce test a permis de montrer une activité antidépressive certaine pour

i) le composé 27.p (racémique trans) avec une dose de 25 mg/kg.

ii) le composé 36.n (racémique trans) avec une dose de 100 mg/kg.

D. Activité anti-inflammatoire

L'activité anti-inflammatoire de composés selon l'invention a été mise en évidence par l'étude de la réduction d'une réaction inflammatoire induite par injection de carragénine sous l'aponévrose plantaire de la patte postérieure du rat (carragénine Sigma type IV, solution de 1% dans du soluté isotonique de NaCl).

Le test utilise un appareil de mesure de volume du type "pléthysmométre" constitué d'une structure en U remplie d'eau, une branche du U étant constituée d'un cylindre destiné à recevoir la patte de l'animal (la patte de l'animal est plongée jusqu'à coïncidence de l'articulation tibio-tarsienne avec un repère de base), l'autre branche étant constituée d'une pipette graduée permettant la mesure de volume en mode différentiel.

Selon le protocole expérimental, au temps "t0" on mesure le volume de la patte de l'animal puis on administre le produit de traitement à étudier (les animaux témoins recevant le véhicule d'administration dans les mêmes conditions). Au temps T0 + 1 heure, on injecte sous l'aponévrose plantaire de chaque animal 0,05 ml de la solution de carragénine. Le volume de la patte de l'animal est mesuré 3, 4 et 5 heures après injection de la carragénine.

L'activité anti-inflammatoire est calculée en pourcentage de réduction de l'oedème chez les rats traités par rapport aux rats témoins.

Ce test a permis de montrer une activité anti-inflammatoire certaine pour les composés 27.p (racémique trans) et 27.p (racémique cis) avec une dose de 15 mg/kg. Les résultats correspondants sont donnés dans le tableau 6.

Tableau 6

| Composé | % d'inhibitions de l'oedème/témoin | | |
|---|---|---|---|
| | à 3 h | à 4 h | à 5 h |
| 27.p (cis) | 35* | 39** | 43** |
| 27.p (trans) | 40** | 40*** | 39** |

Ainsi donc les essais pharmacologiques montrés ci-dessus permettent d'envisager l'application thérapeutique des composés de formule (I) selon l'invention et de leurs sels pharmaceutiquement acceptables notamment comme antalgiques/analgésiques, antidépresseurs et anti-inflammatoires. Plus particulièrement l'invention concerne également des compositions pharmaceutiques contenant un composé de formule (I) ou son sel d'addition d'acide pharmaceutiquement acceptable associé à un véhicule ou excipient pharmaceutiquement acceptable. Les médicaments peuvent être présentés par exemple sous forme de comprimés, d'ampoules injectables, de suppositoires ou de gélules à des doses variant de 10 mg à 500 mg par prise.

## Revendications

**1.** Composés répondant à la formule générale (I)

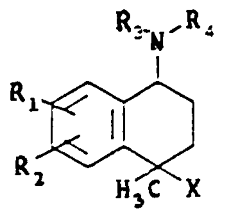

(I)

dans laquelle:

- X — représente un noyau aromatique, notamment phényle, naphtyle, α ou β-thiényle, pouvant porter un à deux substituants choisis parmi halogéno, hydroxy, alcoxy en $C_1$ à $C_8$;
- $R_1$ — représente un atome d'hydrogène ou un halogène, un groupe hydroxy, un groupe alcoxy en $C_1$ à $C_8$, un groupe alkyle linéaire ou ramifié en $C_1$ à $C_4$ éventuellement halogéné, occupant l'une des positions 5, 6 ou 7, ou encore un groupe méthylènedioxy, occupant les positions 5 et 6 ou 6 et 7;
- $R_2$ — représente un atome d'hydrogène ou un halogène, un groupe hydroxy, un groupe alcoxy en $C_1$ à $C_8$, un groupe alkyle linéaire ou ramifié en $C_1$ à $C_4$ éventuellement halogéné, occupant l'une des autres positions 5, 6 ou 7; et
- $R_3$ et $R_4$ — représentent chacun un atome d'hydrogène, un groupe méthyle ou un groupe alkyle linéaire ou ramifié en $C_1$ à $C_n$, n étant 2 ou plus, éventuellement halogéné, hydroxylé ou aminé, ou bien $R_3$ et $R_4$, pris ensemble avec l'atome d'azote auquel ils sont rattachés, forment un hétérocycle saturé, éventuellement substitué, à cinq ou six atomes dont un ou deux sont des hétéroatomes choisis parmi azote, oxygène ou soufre.

**2.** Composés selon la revendication 1, caractérisés en ce que dans la formule (I), X représente un noyau phényle pouvant porter un ou deux substituants choisis parmi les radicaux halogéno, hydroxy, alcoxy en $C_1$ à $C_8$.

**3.** Composés selon la revendication 2, caractérisés en ce que dans la formule (I), X représente un noyau phényle portant un à deux atomes d'halogène, notamment un atome de fluor en position 4, ou un groupe méthoxy.

**4.** Composés selon l'une quelconque des revendications 1 à 3, caractérisés en ce que dans la formule (I), $R_1$ représente un atome d'hydrogène ou un groupe tertiobutyle et $R_2$ un atome d'hydrogène.

**5.** Composés selon l'une quelconque des revendications 1 à 4, caractérisés en ce que dans la formule (I), $R_3$ et $R_4$ représentent chacun un atome d'hydrogène.

**6.** Composés selon l'une quelconque des revendications 1 à 4, caractérisés en ce que dans la formule (I), $R_3$ représente un atome d'hydrogène et $R_4$ représente un groupe méthyle ou un groupe de formule $-(CH_2)_n-OH$ dans laquelle n est 2 ou plus.

**7.** Composés selon l'une quelconque des revendications 1 à 4, caractérisés en ce que, dans la formule (I), $R_3$ et $R_4$ représentent chacun un groupe méthyle ou $R_3$ et $R_4$ pris ensemble avec l'atome d'azote auquel ils sont rattachés forment un groupe de formule

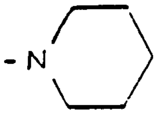 , 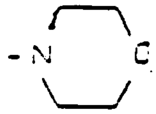 , 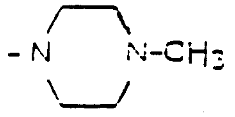 , 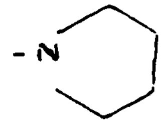 , 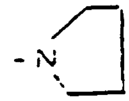

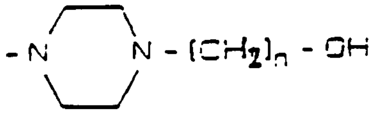 où n = 2 ou plus et/ou OH peut être sous forme ester, uréthanne pour n=2

**8.** Composés selon l'une quelconque des revendications 1 à 4, caractérisés en ce que dans la formule (I), $R_3$et $R_4$ pris ensemble avec l'atome d'azote avec lequel ils sont attachés forment un groupe de formule:

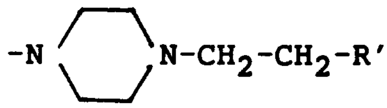

où R' représente un radical OH, un radical acétyle ou un radical uréthanne notamment phényluréthanne ou naphtyluréthanne.

**9.** Composés selon la revendication 1, répondant à la formule (I), dans laquelle X représente un noyau phényle portant un à deux atomes de chlore ou de fluor ou un groupe méthoxy, $R_1$ représente un groupe méthoxy ou un atome de chlore, $R_2$ et $R_3$ représentent chacun un atome d'hydrogène et $R_4$ représente un groupe méthyle ou un groupe de formule $-CH_2-CH_2-OH$.

**10.** Composés selon la revendication 1, répondant à la formule (I), dans laquelle X représente un noyau phényle portant un à deux atomes de chlore ou de fluor ou un groupe méthoxy, $R_1$ représente un groupe tertiobutyle, $R_2$ représente un atome d'hydrogène et $R_3$ et $R_4$ ensemble avec l'atome d'azote auquel ils sont rattachés forment un groupe de formule

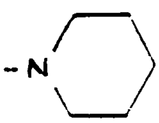 , 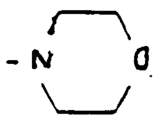 , 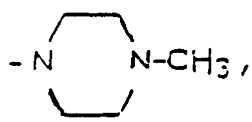 , 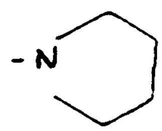 , 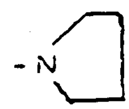

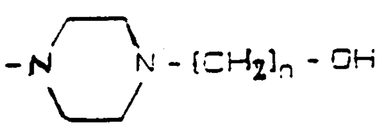 où n = 2 ou plus et/ou OH peut être sous forme ester, uréthanne pour n=2

**11.** Composés selon la revendication 1, répondant à la formule (I), dans laquelle X représente un noyau phényle portant un à deux atomes de chlore ou de fluor ou un groupe méthoxy, $R_1$ représente un groupe méthylène-dioxy fixé en position 6 et 7, $R_2$ représente un atome d'hydrogène et $R_3$ et $R_4$ représentent chacun un atome d'hydrogène, un groupe méthyle ou un groupe de formule $CH_2)_n$-OH, ou bien $R_3$ et $R_4$ pris ensemble avec l'atome d'azote auquel ils sont rattachés forment un groupe de formule:

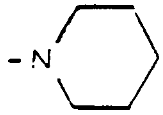 , 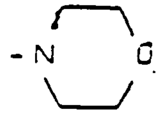 , 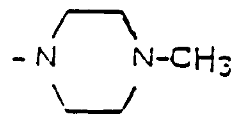 , 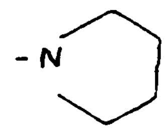 , 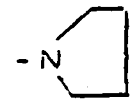

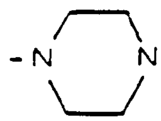 $-(CH_2)_n-OH$ où n = 2 ou plus et/ou OH peut être sous forme ester, uréthanne pour n=2

**12.** Sels d'addition d'acide d'un composé conforme à l'une quelconque des revendications 1 à 11.

**13.** Procédé de préparation d'un composé selon l'une quelconque des revendications 1 à 12, caractérisé en ce que

- l'on prépare un halogénure de méthyl-1-phényl-1 tétraline de formule (IV)

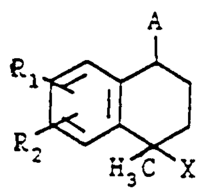

**(IV)**

dans laquelle $R_1$, $R_2$ et X ont la même signification que dans la formule (I) et A représente un halogène, à partir d'une méthyl-1-phényl-1 tétraline de formule (III)

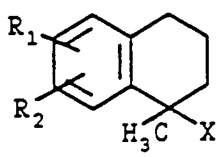

**(III)**

dans laquelle $R_1$, $R_2$ et X ont la même signification que dans la formule (I);

- l'on fait réagir le composé de formule (IV) sur une amine de formule

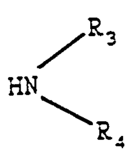

**(V)**

dans laquelle $R_3$ et $R_4$ ont la même signification que dans la formule (I) pour obtenir un mélange des deux diastéréoisomères du composé de formule (I).

**14.** Procédé selon la revendication 13, caractérisé en ce que l'on fait réagir l'halogénure de formule (IV) sur l'amine de formule (V) en solution organique (toluène, acétone, $CH_2Cl_2$, $CHCl_3$) pendant une durée supérieure à 24 h à température ambiante ou quelques heures au reflux du solvant.

**15.** Procédé selon l'une des revendications 13 et 14, caractérisé en ce que l'halogénure de formule (IV) est obtenu par halogénation d'un composé de formule (III) effectué par une halogénosuccinimide au reflux de $CCl_4$ en présence de péroxyde de benzoyle.

**16.** Procédé selon l'une des revendications 13 et 14, caractérisé en ce que l'halogénure de formule (IV) est

obtenu par:

- oxydation benzylique d'un dérivé de formule (III) pour obtenir la tétralone correspondante de formule (VI)

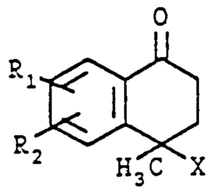

(VI)

dans laquelle $R_1$, $R_2$ et X ont la même signification que dans la formule (I);

- réduction du composé de formule (VI) par un hydrure (notamment $N_aBH_4$ ou $LiAlH_4$) pour obtenir le tétralol correspondant de formule (VII)

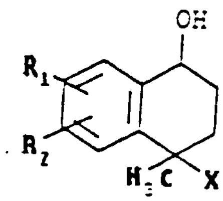

(VII)

dans laquelle $R_1$, $R_2$ et X ont la même signification que dans la formule (I);

- substitution par un chlorure, en présence de chlorure de thionyle pour donner l'halogénure de formule (IV).

**17.** Procédé de préparation d'un composé de formule (I) selon l'une des revendications 1 à 12, caractérisé en ce que l'on oxyde selon une oxydation benzylique un dérivé de formule (III) pour obtenir la tétralone correspondante de formule

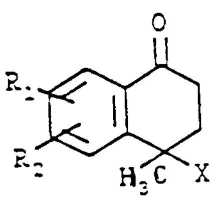

(VI)

dans laquelle $R_1$, $R_2$ et X ont la même signification que dans la formule (I)
et en ce que l'on fait réagir le composé de formule (VI) sur une amine de formule (V) pour obtenir un dérivé azoté (oxime si $R_3$ et $R_4$ représentent chacun un atome d'hydrogène, imine si $R_3$ ou $R_4$ représente un atome d'hydrogène et énamine si ni $R_3$ ni $R_4$ ne représente un atome d'hydrogène) que l'on réduit par un hydrure (notamment $NaBH_4$ ou $LiAlH_4$) ou par l'hydrogène en présence d'un catalyseur, pour obtenir l'amino-4-méthyl-1 tétraline correspondante qui peut être une amine primaire ($R_3$ et $R_4$ représentant chacun un atome d'hydrogène), une amine secondaire ($R_3$ ou $R_4$ représenteun atome d'hydrogène) ou une amine tertiaire (ni $R_3$ ni $R_4$ ne représente un atome d'hydrogène).

**18.** Procédé selon l'une des revendications 13 à 17, caractérisé en ce que le composé de formule (III) est obtenu par solvolyse en milieu acide dans un solvant organique, notamment le benzène, le toluène ou le dichlorométhane ($CH_2Cl_2$), d'un composé de formule (II)

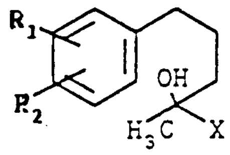

(II)

dans laquelle $R_1$, $R_2$ et X ont la même signification que dans la formule (I).

**19.** Procédé selon la revendication 18, caractérisé en ce que la solvolyse est réalisée en milieu acide constitué par un acide protique, notamment l'acide sulfurique, l'acide trifluoroacétique, la solvolyse étant effectuée au reflux du solvant ou à température ambiante.

**20.** Procédé selon la revendication 18, caractérisé en ce que la solvolyse est réalisée en milieu acide constitué par un acide de Lewis, de préférence $AlCl_3$.

**21.** Composition pharmaceutique contenant un composé selon l'une des revendications 1 à 12, ainsi qu'un véhicule ou excipient pharmaceutiquement acceptable.

**22.** Médicament contenant un composé conforme à l'une des revendications 1 à 12 pour le traitement des algies.

**23.** Médicament contenant un composé conforme à l'une des revendications 1 à 12 pour le traitement des états dépressifs.

**24.** Médicament contenant un composé conforme à l'une des revendications 1 à 12 pour le traitement des inflammations.

**Revendications pour l'Etat contractant suivant : ES**

**1.** Procédé de préparation d'un composé répondant à la formule générale (I)

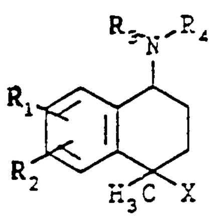

(I)

dans laquelle:

X — représente un noyau aromatique, notamment phényle, naphtyle, α ou β-thiényle pouvant porter un à deux substituants choisis parmi halogéno, hydroxy, alcoxy en $C_1$ à $C_8$ trifluorométhyle;

$R_1$ — représente un atome d'hydrogène ou un halogène, un groupe hydroxy, un groupe alcoxy en $C_1$ à $C_8$, un groupe alkyle linéaire ou ramifié en $C_1$ à $C_4$ éventuellement halogéné, occupant l'une des positions 5, 6 ou 7, ou encore un groupe méthylènedioxy occupant les positions 5 et 6 ou 6 et 7;

$R_2$ — représente un atome d'hydrogène ou d'halogène, un groupe hydroxy, un groupe alcoxy en $C_1$ à $C_8$, un groupe alkyle linéaire ou ramifié en $C_1$ à $C_4$ éventuellement halogéné, occupant l'une des autres positions 5, 6 ou 7; et

$R_3$ et $R_4$ — représentent chacun un atome d'hydrogène, un groupe alkyle linéaire ou ramifié en $C_1$ à $C_n$, n étant 2 ou plus, éventuellement halogéné, hydroxylé ou aminé, ou bien $R_3$ et $R_4$, pris ensemble avec l'atome d'azote auquel ils sont rattachés forment un hétérocycle saturé, éventuellement substitué, à cinq ou six atomes dont un ou deux sont des hétéroatomes choisis parmi azote, oxygène ou soufre,

caractérisé en ce que

- l'on prépare un halogénure de méthyl-1-phényl-1-tétraline de formule (IV)

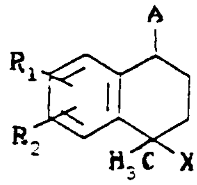

(IV)

dans laquelle $R_1$, $R_2$ et X ont la même signification que dans la formule (I) et A représente un halogène, à partir d'une méthyl-1-phényl-1 tétraline de formule (III)

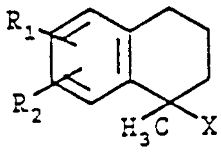

(III) (III)

dans laquelle $R_1$, $R_2$ et X ont la même signification que dans la formule (I);

- l'on fait réagir le composé de formule (IV) sur une amine de formule

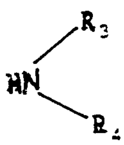

(V)

dans laquelle $R_3$ et $R_4$ ont la même signification que dans la formule (I) pour obtenir un mélange des deux diastéréoisomères du composé de formule (I).

2. Procédé selon la revendication 1, caractérisé en ce que l'on fait réagir l'halogénure de formule (IV) sur l'amine de formule (V) en solution organique (toluène, acétone, $CH_2Cl_2$, $CHCl_3$) pendant une durée supérieure à 24 h à température ambiante ou quelques heures au reflux du solvant.

3. Procédé selon l'une des revendications 1 et 2, caractérisé en ce que l'halogénure de formule (IV) est obtenu par halogénation d'un composé de formule (III) effectué par une halogénosuccinimide au reflux de $CCl_4$ en présence de péroxyde de benzoyle.

4. Procédé selon l'une des revendications 1 et 2, caractérisé en ce que l'halogénure de formule (IV) est obtenu par:
   - oxydation benzylique d'un dérivé de formule (III) pour obtenir la tétralone correspondante de formule (VI)

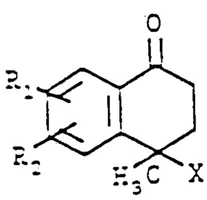

(VI)

dans laquelle $R_1$, $R_2$ et X ont la même signification que dans la formule (I);

   - réduction du composé de formule (VI) par un hydrure (notamment $NaBH_4$ ou $LiAlH_4$) pour obtenir le tétralol correspondant de formule (VII)

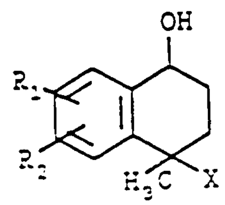

(VII)

dans laquelle $R_1$, $R_2$ et X ont la même signification que dans la formule (I);

- substitution par un chlorure, en présence de chlorure de thionyle pour donner l'halogénure de formule (IV).

5. Procédé de préparation d'un composé répondant à la formule générale (I)

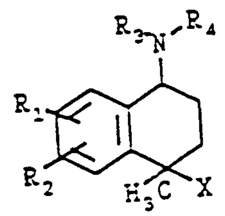

(I)

dans laquelle X, $R_1$, $R_2$, $R_3$ et $R_4$ ont la même signification que celle donnée à la revendication 1, caractérisé en ce que l'on oxyde selon une oxydation benzylique un dérivé de formule (III) pour obtenir la tétralone correspondante de formule

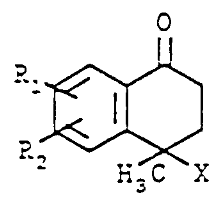

(VI)

dans laquelle $R_1$, $R_2$ et X ont la même signification que dans la formule (I)

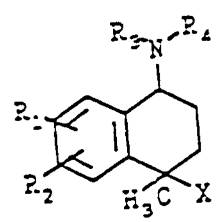

(I)

et en ce que l'on fait réagir le composé de formule (VI) sur une amine de formule (V) pour obtenir un dérivé azoté (oxime si $R_3$ et $R_4$ représentent chacun un atome d'hydrogène, imine si $R_3$ ou $R_4$ représente un atome d'hydrogène et énamine si ni $R_3$ ni $R_4$ ne représente un atome d'hydrogène) que l'on réduit par un hydrure (notamment $NaBH_4$ ou $LiAlH_4$) ou par l'hydrogène en présence d'un catalyseur, pour obtenir l'amino-4-méthyl-1 tétraline correspondante qui peut être une amine primaire ($R_3$ et $R_4$ représentant chacun un atome d'hydrogène), une amine secondaire ($R_3$ ou $R_4$ représenteun atome d'hydrogène) ou une amine tertiaire (ni $R_3$ ni $R_4$ représente un atome d'hydrogène).

6. Procédé selon l'une des revendications 1 à 5, caractérisé en ce que le composé de formule (III) est obtenu par solvolyse en milieu acide dans un solvant organique, notamment le benzène, le toluène ou le dichlorométhane ($CH_2Cl_2$), d'un composé de formule (II)

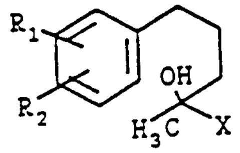

(II)

dans laquelle $R_1$, $R_2$ et X ont la même signification que dans la formule (I).

**7.** Procédé selon la revendication 6, caractérisé en ce que la solvolyse est réalisée en milieu acide constitué par un acide protique, notamment l'acide sulfurique, l'acide trifluoroacétique, la solvolyse étant effectuée au reflux du solvant ou à température ambiante.

**8.** Procédé selon la revendication 6, caractérisé en ce que la solvolyse est réalisée en milieu acide constitué par un acide de Lewis, de préférence $AlCl_3$.

**9.** Procédé selon l'une des revendications 1 à 8, pour préparer un composé de formule (I) dans lequel le groupe $NR_3R_4$ se présente sous la formule

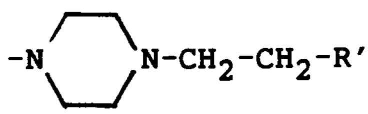

où

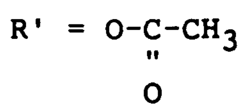

caractérisé en ce que l'amine (V) est obtenue par action de l'anhydride de l'acide acétique sur la pipérazine-éthanol en milieu organique et en présence de trace de 4-diméthylamino-pyridine à température ambiante pendant 24 h.

**10.** Procédé selon l'une des revendications 1 à 9 pour préparer un composé de formule I dans lequel le groupe $NR_3R_4$ se présente sous la forme

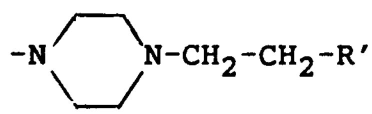

où R' est un radical uréthanne, notamment phényluréthanne ou naphtyluréthanne,
caractérisé en ce que,
l'uréthanne est obtenu par action d'un isocyanate sur le pipérazine-éthanol dans un solvant organique anhydre à température ambiante pendant 24 h.

**11.** Utilisation d'un composé obtenu par mise en oeuvre du procédé selon l'une des revendications 1 à 10 à la préparation de médicament pour le traitement des algies.

**12.** Utilisation d'un composé obtenu par mise en oeuvre du procédé selon l'une des revendications 1 à 10 à la préparation de médicament pour le traitement des états dépressifs.

**13.** Utilisation d'un composé obtenu par mise en oeuvre du procédé selon l'une des revendications 1 à 10 à la préparation de médicament pour le traitement des inflammations.

FIG.1

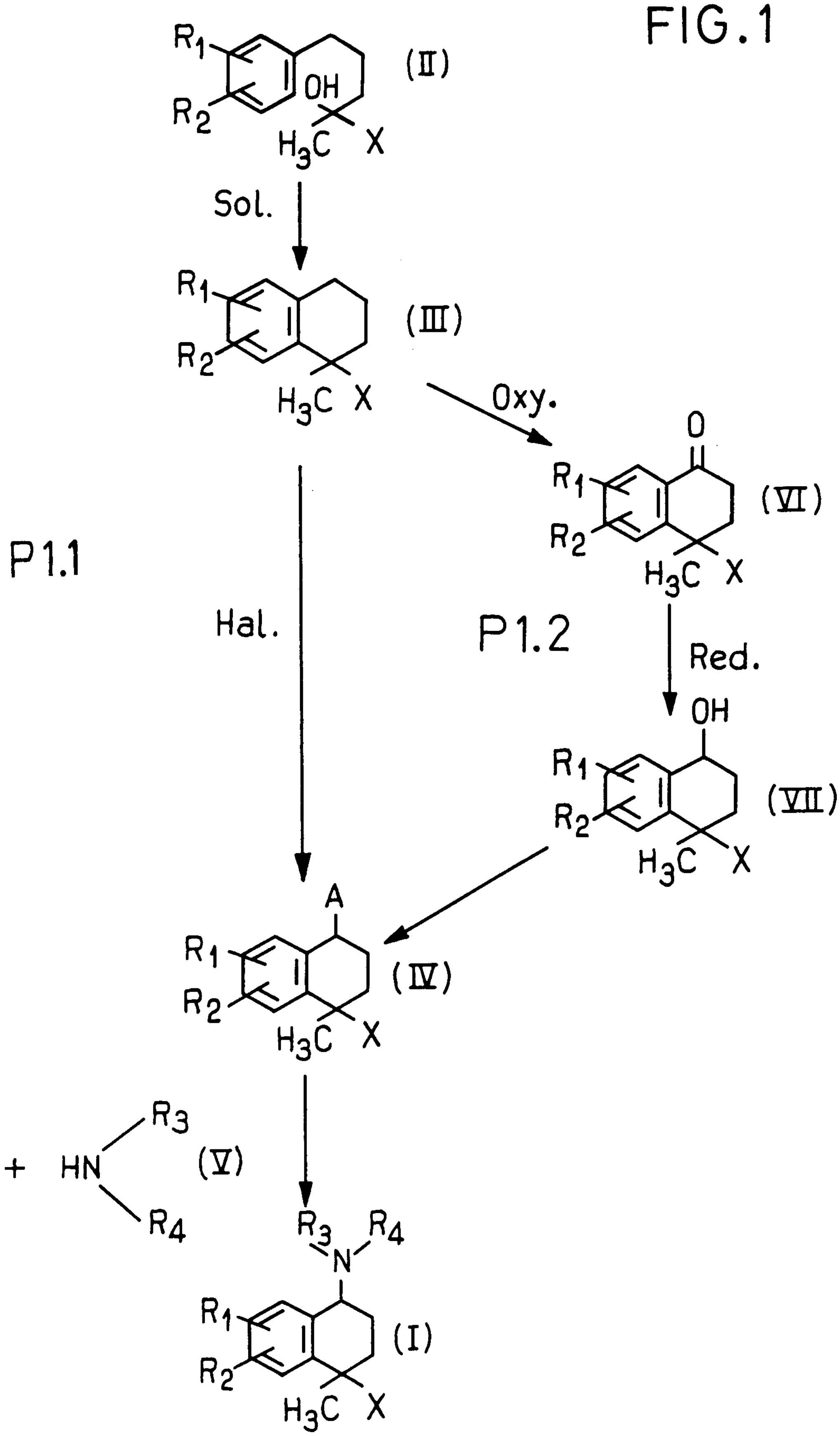

FIG_2

P2

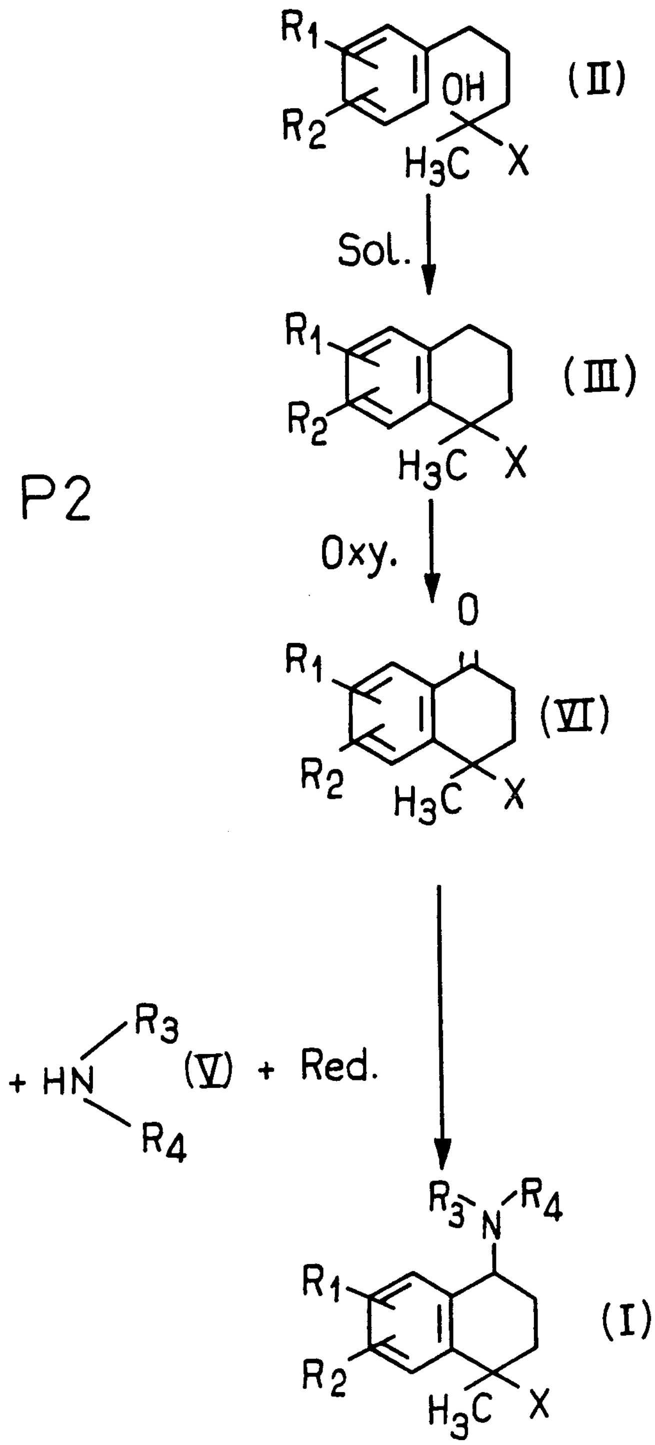

Office européen des brevets

# RAPPORT DE RECHERCHE EUROPEENNE

Numero de la demande

EP 91 20 1972

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | EP-A-0 028 901 (PFIZER)<br>* revendications; exemples * | 1-24 | C07D295/08<br>A61K31/495<br>C07D295/12<br>C07D295/02<br>C07D295/12<br>C07C217/74<br>C07C211/42<br>C07C215/42<br>C07D333/20<br>C07D317/70<br>A61K31/535<br>A61K31/135 |
| A | EP-A-0 030 081 (PFIZER)<br>* revendications; exemples * | 1-24 | |
| A | GB-A-1 301 434 (PFIZER)<br>*en entier* | 1-24 | |
| A | DE-C-946 058 (SCHERING)<br>*en entier* | 1-24 | |
| D,A | WO-A-8 904 820 (LABORATOIRES LUCIEN)<br>*en entier* | 1-24 | |
| | | | **DOMAINES TECHNIQUES RECHERCHES (Int. Cl.5 )** |
| | | | C07D<br>C07C |

Le présent rapport a été établi pour toutes les revendications

| Lieu de la recherche | Date d'achèvement de la recherche | Examinateur |
|---|---|---|
| LA HAYE | 05 NOVEMBRE 1991 | f.m. helps |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant